**Europäisches Patentamt**

**European Patent Office**

**Office européen des brevets**

(19)

(11) Veröffentlichungsnummer : **0 474 040 B1**

## (12) EUROPÄISCHE PATENTSCHRIFT

(45) Veröffentlichungstag der Patentschrift :
**06.04.94 Patentblatt 94/14**

(51) Int. Cl.⁵ : **A23L 2/38,** A23L 2/40,
A61K 9/46

(21) Anmeldenummer : **91114031.7**

(22) Anmeldetag : **22.08.91**

(54) **Brausekomponente und Verfahren zu ihrer Herstellung.**

(30) Priorität : **04.09.90 DE 4027927**

(43) Veröffentlichungstag der Anmeldung :
**11.03.92 Patentblatt 92/11**

(45) Bekanntmachung des Hinweises auf die
Patenterteilung :
**06.04.94 Patentblatt 94/14**

(84) Benannte Vertragsstaaten :
**AT BE CH DE DK ES FR GB GR IT LI LU NL SE**

(56) Entgegenhaltungen :
**WO-A-87/05507**
**DE-A- 3 635 864**
**DE-B- 2 553 444**
**DE-B- 2 842 822**

(73) Patentinhaber : **BAYER AG**
**D-51368 Leverkusen (DE)**

(72) Erfinder : **Maasz, Joachim, Dr.**
**Hüschelrath 4**
**W-5653 Leichlingen (DE)**
Erfinder : **Fritsch, Christian, Dr.**
**Wiesengrundweg 7**
**W-8520 Erlangen (DE)**
Erfinder : **Gräwingholt, Werner**
**Unter Buschweg 114**
**W-5000 Köln 50 (DE)**
Erfinder : **Streuff, Bernhard, Dr.**
**Asterweg 23**
**W-5632 Wermelskirchen (DE)**
Erfinder : **Frank, Georg, Dr.**
**Henselweg 17**
**W-5600 Wuppertal 1 (DE)**

EP 0 474 040 B1

**Beschreibung**

Die vorliegende Erfindung betrifft eine saure Brausekomponente zur Direkttablettierung von Brausetabletten sowie Verfahren zu ihrer Herstellung.

Brausetabletten bestehen üblicherweise aus dem Wirkstoff, einem Brausesatz und gegebenenfalls weiteren Hilfsstoffen. Der Brausesatz besteht aus einer sauren Komponente wie z.B. Citronen- oder Weinsäure oder einer anderen physiologisch unbedenklichen Säure und einer alkalischen Komponente wie z.B. Natrium-, Kalium- oder Calciumcarbonat oder Natrium- und Kalium-bicarbonat, bei deren Reaktion miteinander gasförmiges Kohlendioxid entsteht. Als Alternative zu den genannten Säuren können auch deren saure Salze oder Gemische der Säuren mit den Salzen verwendet (vgl. DOS 1 962 791) werden.

Da sowohl die saure als auch die basische Komponente des Brausesatzes sehr schlecht direkttablettiert werden können, ist in der Regel eine vorherige Granulation eines Teils dieser Komponenten oder aller Komponenten der Brausetablette erforderlich (vgl. DE 25 53 444, DE 2 216 072 oder EP 233 853). Die vorausgehende Granulation einzelner oder aller Komponenten einer Brausetablette ist jedoch mit hohem apparativem, finanziellem und zeitlichem Aufwand verbunden. Oft müssen neben der Vorgranulation noch zusätzlich Bindemittel eingesetzt werden. Deshalb wurde in der Vergangenheit wiederholt eine Direkttablettierung der Ausgangskomponenten versucht, z.B. durch Einsatz spezieller Bindemittel (vgl. EP 219 337). Solche speziellen Bindemittel wie z.B. Dextrose stellen eine für die Wirksamkeit der Brausetablette unnötige Komponente dar. Neben der Vergrößerung der Tablette hat sie den Nachteil einen kalorischen Nährwert zu besitzen und ist als Kohlenhydrat für Diabetiker ungeeignet, was bei den erforderlichen Mengen von 200-400 mg/Tablette nicht zu vernachlässigen ist.

Es wurde auch versucht, eine Direkttablettierung durch Sprühtrocknung von Natriumbicarbonat als basische Komponente zu erreichen (vgl. Saleh et al., Int. J. Pharm. 45 (1988) 19-26). Dies gelang jedoch nur unter Zusatz weiterer Hilfsstoffe wie z.B. von Bindemittel. Als weitere Nachteile des dort beschriebenen Prozesses sind zu nennen die unwirtschaftliche und aufwendige Prozeßführung. Aufgrund der geringen Löslichkeit von Natriumbicarbonat in Wasser kann nur eine etwa 7 %ige Lösung von Natriumbicarbonat sprühgetrocknet werden. Die Sprühtrocknung von höher konzentrierten Suspensionen ist für Natriumbicarbonat wenig sinnvoll aufgrund seiner thermischen Zersetzung bei Temperaturen oberhalb 50°C.

Es wurde gefunden, daß die neuen sauren Brausekomponenten, die durch Sprühtrocknung hergestellt wurden, diese nachteiligen Tablettiereigenschaften nicht mehr besitzen. Mit ihnen ist eine Direkttablettierung von Brausetabletten in einfacher Weise und ohne zusätzliche Bindemittel möglich.

Gegenstand der Erfindung ist eine neue saure sprühgetrocknete Brausekomponente, die im wesentlichen kugelförmig ist und ein saures Salz der Wein- und/oder Citronensäure enthält. Von besonderem Interesse sind erfindungsgemäße Brausekomponenten, die im wesentlichen Mono- oder Dinatriumcitrat, Mono- oder Dikaliumcitrat, Mono- oder Diammoniumcitrat, Mononatrium-, Monokalium- oder oder Monoammoniumtartrat oder Mischungen dieser Salze enthalten. Besonders bevorzugt sei das Mononatriumcitrat genannt.

Besonders geeignet sind saure Brausekomponenten in Form von kugelförmigen Einzelpartikeln mit einer mittleren Teilchengröße von 70 bis 250 µm und einem Schüttvolumen von 110 bis 350 ml/100 g.

Besonders bevorzugt ist eine mittlere Teilchengröße von 100 bis 190 µm und ein Schüttvolumen von 130 bis 270 ml/100 g.

Die Erfindung betrifft auch ein Verfahren zur Herstellung der direkttablettierbaren sauren Brausekomponente, dadurch gekennzeichnet, daß saure Salze der Wein- oder Citronensäure sprühgetrocknet werden. Hierbei werden übliche Sprühtrocknungsverfahren z.B. kontinuierliche oder diskontinuierliche Sprühtrocknung im Gleichstrom-, Gegenstrom- oder Mischstromverfahren, Sprühagglomeration, Sprühtrocknung in oder auf eine Pulverwirbelschicht und ähnliche Verfahren angewendet.

In einer bevorzugten Ausführungsform wird im erfindungsgemäßen Sprühtrocknungsverfahren zunächst eine Lösung oder Suspension der genannten sauren Salze in einem geeigneten Lösungsmittel oder Lösungsmittelgemisch als Speiseflüssigkeit hergestellt, derart, daß der Feststoffanteil 10 bis 70 %, bevorzugt 30 bis 50 %, der fertigen Speiseflüssigkeit beträgt und dann diese Speiseflüssigkeit in einem üblichen Sprühtrockner bei einer Lufteintrittstemperatur von 100 bis 250°C, bevorzugt 120 bis 210°C, und einer Luftaustrittstemperatur von 40 bis 170°C, bevorzugt 50 bis 140°C, getrocknet.

Die so hergestellte saure Brausekomponente mit im wesentlichen kugelförmigen Partikeln läßt sich in vorteilhafter Weise direkt zu stabilen Brausetabletten tablettieren, ohne daß zusätzliche Bindemittel erforderlich sind. Die erfindungsgemäße saure Brausekomponente, deren Einzelpartikel im wesentlichen in Kugelform erhalten werden, die je nach Trocknungsparametern unterschiedlich große Hohlräume enthalten können, sind in ihren Tablettierungseigenschaften den bekannten Granulaten saurer Brausekomponenten überlegen. Insbesondere ist die Härte und Abriebfestigkeit von Brausetabletten, die mit der erfindungsgemäßen Brausekomponente hergestellt wurden, wesentlich besser als die von vergleichbaren Brausetabletten, die durch Vorgranulierung der sauren Brausekomponente hergestellt wurden. Überraschenderweise wird durch die verbesserte Härte und Abriebfestigkeit der erfindungsgemäßen Zubereitungen die Auflösungszeit der Brausetablette nicht verlängert sondern sogar noch verkürzt.

Die vorliegende Erfindung ist geeignet, das seit langem bestehende Befürfnis nach einer einfachen Herstellung von stabilen Brausetabletten mit vorteilhaften Eigenschaften zu ermöglichen. Im einzelnen seien folgende Vorteile genannt:

a) Die kugelförmige saure Brausekomponente kann in einfacher Weise, gegebenenfalls kontinuierlich, und ohne Verwendung unerwünschter Hilfsstoffe hergestellt werden.

b) Durch Verwendung dieser sauren Komponente können Brausetabletten direkt, d.h. ohne vorherige Granulation einzelner Bestandteile, tablettiert werden.

c) Wegen der hohen Stabilität der sauren Brausekomponente kann die Tablettierung bei höheren Drucken und mit höherer Geschwindigkeit, z.B. auch auf Hochleistungspressen, durchgeführt werden.

d) Bei der Herstellung der Brausetabletten sind keine zusätzlichen Bindemittel, Lösungsvermittler oder Zerfallsbeschleuniger notwendig.

e) Die Härte und Abriebfestigkeit der erfindungsgemäß hergestellten Brausetabletten ist verbessert, was zu einer höheren mechanischen Stabilität der Tabletten führt und Verpackung und Transport vereinfacht.

f) Trotz größerer Härte ist die Auflösungszeit der erfindungsgemäß hergestellten Brausetabletten verkürzt.

Die erfindungsgemäße saure Brausekomponente kann sehr vielseitig eingesetzt werden, z.B. im Bereich der Pharmazie und der Kosmetik, aber auch bei Lebensmitteln und Produkten des Veterinärbereichs. Sie ist einsetzbar in allen Formen, in denen Brausezubereitungen Verwendung finden, z.B. in Form von Tabletten, Granulaten oder Pulvern. Besonders bevorzugt ist die pharmazeutische Anwendung. Hierbei läßt sich die erfindungsgemäße Brausekomponente mit allen Arten von Wirkstoffen, die in einer Brausezubereitung appliziert werden können, kombinieren. Von besonderem Interesse sind Analgetika wie z.B. Acetylsalicylsäure, Paracetamol, Ibuprofen, Ketoprofen und Naproxen jeweils in racemischer Form oder in Form ihrer reinen Enantiomeren oder Antacida, Vitamine, Psychotherapeutika und andere oral applizierbare Wirkstoffe, insbesondere Wirkstoffe zur Behandlung von Husten, Magenerkrankungen und Erkältungskrankheiten.

Die nachfolgenden Ausführungsbeispiele sollen den Erfindungsgegenstand näher erläutern ohne ihn einzuschränken.

Beispiele

Beispiel 1

40,8 kg Mononatriumcitrat werden in 59,2 kg entmineralisiertem Wasser suspendiert und anschließend bei 210°C Lufteintrittstemperatur und 140°C Luftaustrittstemperatur sprühgetrocknet. Das sprühgetrocknete Natriumcitrat besitzt eine mittlere Teilchengröße von 125 µm und ein Schüttvolumen von 260 ml/100 g.

Beispiel 2

40,8 kg Mononatriumcitrat werden unter Erwärmen in 59,2 kg entmineralisiertem Wasser gelöst und bei 150°C Lufteintrittstemperatur und 100°C Luftaustrittstemperatur getrocknet. Das sprühgetrocknete Natriumcitrat besitzt eine mittlere Teilchengröße von 120 µm und ein Schüttvolumen von 162 ml/100 g.

Beispiel 3

37,3 kg Citronensäure werden in 32,1 kg entmineralisiertem Wasser gelöst und durch Zugabe von 17,3 kg 45 %iger Natronlauge zu Mononatriumcitrat umgesetzt. Es ergibt sich ein Feststoffgehalt von 41,6 % Mononatriumcitrat. Nach Sprühtrocknung bei einer Lufteintrittstemperatur von 120°C und einer Luftaustrittstemperatur von 60°C erhält man sprühgetrocknetes Natriumcitrat mit einer mittleren Teilchengröße von 100 µm und einem Schüttvolumen von 158 ml/100 g.

Beispiel 4

37,3 kg Citronensäure werden in 45,1 kg entmineralisiertem Wasser gelöst, durch Zugabe von 24,4 kg 45 %iger Kalilauge zu Monokaliumcitrat umgesetzt und analog Beispiel 3 sprühgetrocknet.

## Beispiel 5

45 kg Mononatriumtartrat werden in 55 kg entmineralisiertem Wasser suspendiert und bei 130°C Lufteingangstemperatur und 70°C Luftausgangstemperatur sprühgetrocknet. Das sprühgetrocknete Mononatriumtartrat besitzt eine mittlere Teilchengröße von 110 µm und ein Schüttvolumen von 145 ml/100 g.

## Beispiel 6

45 kg der erfindungsgemäßen Brausekomponenten, hergestellt nach den Beispielen 1 und 3 werden mit jeweils 12,5 kg Acetylsalicylsäure, 28,5 kg Natriumbicarbonat und 6 kg Natriumcarbonat gemischt und auf einer Tablettenpresse zu Tabletten mit einem Gewicht von 3,2 g verpreßt. Zum Vergleich werden anstelle der erfindungsgemäßen Brausekomponente 45 kg eines Natriumcitrat-Granulates, enthaltend 20 Gew.-% Citronensäure als Bindemittel, verwendet. Die resultierenden Tablettenhärten und Zerfallszeiten zeigen eine deutliche Verbesserung bei Verwendung der erfindungsgemäßen Brausekomponente (s. Tabelle).

| verwendete saure Brausekomponente | mittlere Tabletten-härte | mittlere Zerfalls-zeit |
|---|---|---|
| Mononatriumcitrat hergestellt nach Beispiel 1 | 120 N | 50 s |
| Mononatriumcitrat hergestellt nach Beispiel 3 | 128 N | 55 s |
| Mononatriumcitrat-Granulat mit 20 % Citronensäure | 72 N | 90 s |

## Beispiel 7

51,2 kg Mononatriumcitrat werden in 68,8 kg entmineralisiertem Wasser suspendiert und in einer Sprühagglomerationsanlage bei einer Lufteintrittstemperatur von 125°C und einer Luftaustrittstemperatur von 55°C getrocknet. Die Produkttemperatur im Fließbett beträgt 58°C. Das Produkt hat eine mittlere Teilchengröße von 100 µm und ein Schüttvolumen von 202 ml/100 g. 45 kg dieses sprühagglomerierten Mononatriumcitrats werden mit 12,5 kg Acetylsalicylsäure, 28,5 kg Natriumbicarbonat, 7 kg Ascorbinsäure und 6 kg Natriumcarbonat gemischt und auf einer Tablettenpresse zu Tabletten mit einem Gewicht von 3,2 g verpreßt. Die Tabletten besitzen eine Härte von etwa 118 N und eine Zerfallszeit von etwa 1 Minute.

## Beispiel 8

8,4 kg Ranitidin-Hydrochlorid werden mit 48 kg sprühgetrocknetem Mononatriumtartrat, 57,5 kg Natriumbicarbonat, 2,5 kg Natriumcyclamat, 10 kg mikronisierter Fumarsäure und 8,5 kg Citronen-Limette-Aroma gemischt, die Mischung auf eine relative Gleichgewichtsfeuchte von maximal 10 % konditioniert und zu Tabletten mit einem Durchmesser von 22 mm und einem Gewicht von 2,5 g verpreßt.

## Beispiel 9

10 kg Ibuprofen werden mit 75 kg sprühgetrocknetem Monokaliumcitrat, 35 kg Natriumbicarbonat, 22,5 kg Natriumcarbonat und 2,5 kg Polyvinylpyrrolidon gemischt und zu Tabletten mit einem Durchmesser von 24 mm und einem Gewicht von 3 g verpreßt.

## Beispiel 10

10 kg Acetylcystein werden mit 42,5 kg sprühgetrocknetem Mononatriumcitrat, 15 kg Natriumbicarbonat, 5 kg mikronisierter Adipinsäure, 1,5 kg Polyethylenglykol 6000, 0,5 kg Natriumstearylfumarat und 0,5 kg eines geeigneten Aromastoffs gemischt und zu Tabletten mit einem Durchmesser von 18 mm und einem Gewicht von 1,5 g verpreßt.

Beispiel 11

3 kg Ambroxol-Hydrochlorid werden mit 80 kg sprühgetrocknetem Mononatriumcitrat, 30 kg Natriumbicarbonat, 10 kg mikronisierter Adipinsäure, 5 kg Polyethylenglykol 6000, 1 kg Natriumstearylfumarat und 1 kg eines geeigneten Aromastoffs gemischt und zu Tabletten mit einem Durchmesser von 17 mm und einem Gewicht von 1,3 g verpreßt.

**Patentansprüche**

**Patentansprüche für folgende Vertragsstaaten : AT, BE, CH, LI, DE, DK, FR, GB, IT, LU, NL, SE**

1.  Saure sprühgetrocknete Brausekomponente, die im wesentlichen kugelförmig ist und ein saures Salz der Wein- und/oder Citronensäure enthält.

2.  Brausekomponente gemäß Anspruch 1, die im wesentlichen Mono- oder Dinatriumcitrat, Mono- oder Dikaliumcitrat, Mono- oder Diammoniumcitrat, Mononatrium-, Monokalium- oder Monoammoniumtartrat oder Mischungen dieser Salze enthält.

3.  Brausekomponente gemäß Anspruch 1, enthaltend im wesentlichen ein Salz der Citronensäure.

4.  Brausekomponente gemäß Ansprüchen 1 bis 3, dadurch gekennzeichnet, daß sie im wesentlichen aus kugelförmigen Einzelpartikeln besteht die eine mittlere Teilchengröße von 70 bis 250 $\mu$m und ein Schüttvolumen von 110 bis 350 ml/100 g besitzen.

5.  Brausekomponente gemäß Anspruch 4 mit einer mittleren Teilchengröße von 100 bis 190 $\mu$m und einem Schüttvolumen von 130 bis 270 ml/100 g.

6.  Verfahren zur Herstellung von sauren sprühgetrockneten Brausekomponenten, die direkttablettierbar sind und die im wesentlichen kugelförmige Teilchen aus einem sauren Salz der Wein- und/oder Citronensäure enthalten, dadurch gekennzeichnet, daß die sauren Salze nach üblichen Methoden sprühgetrocknet werden.

7.  Sprühtrocknungsverfahren gemäß Anspruch 6, dadurch gekennzeichnet, daß zunächst eine Lösung oder Suspension der sauren Salze der wein- und/oder Citronensäure in einem geeigneten Lösungsmittel oder Lösungsmittelgemisch als Speiseflüssigkeit hergestellt wird die einen Feststoffanteil von 10 bis 70 % enthält und dann diese Speiseflüssigkeit in einem Sprühtrockner bei einer Lufteintrittstemperatur von 100 bis 250°C und einer Luftaustrittstemperatur von 40 bis 170°C getrocknet wird.

8.  Verfahren gemäß Anspruch 6, dadurch gekennzeichnet, daß die eingesetzte Speiseflüssigkeit 30 bis 50 % Feststoffanteile enthält.

9.  Verfahren gemäß Anspruch 6, dadurch gekennzeichnet, daß die Sprühtrocknung bei einer Lufteintrittstemperatur von 120 bis 210°C und einer Luftaustrittstemperatur von 50 bis 140°C durchgeführt wird.

10. Verfahren gemäß Ansprüchen 6 bis 8, dadurch gekennzeichnet, daß die Lösung oder Suspension der sauren Salze zusammen mit gegebenenfalls vorhandenen weiteren Zuschlagstoffen sprühgetrocknet wird.

11. Verwendung von Brausekomponenten gemäß Anspruch 1 zur Herstellung von Brausezubereitungen in Form von Brausetabletten, Brausegranulaten oder Brausepulvern.

12. Verfahren zur Herstellung von Brausetabletten, dadurch gekennzeichnet, daß man die Brausekomponente gemäß Anspruch 1 nach üblichen Methoden direkttablettiert.

**Patentansprüche für folgende Vertragsstaaten : ES, GR**

1. Verfahren zur Herstellung von sauren sprühgetrockneten Brausekomponenten, die direkttablettierbar sind und die im wesentlichen kugelförmige Teilchen aus einem sauren Salz der Wein- und/oder Citronensäure enthalten, dadurch gekennzeichnet, daß die sauren Salze nach üblichen Methoden sprühgetrocknet werden.

2. Sprühtrocknungsverfahren gemäß Anspruch 1, dadurch gekennzeichnet, daß zunächst eine Lösung oder Suspension der sauren Salze der Wein- und/oder Citronensäure in einem geeigneten Lösungsmittel oder Lösungsmittelgemisch als Speiseflüssigkeit hergestellt wird die einen Feststoffanteil von 10 bis 70 % enthält und dann diese Speiseflüssigkeit in einem Sprühtrockner bei einer Lufteintrittstemperatur von 100 bis 250°C und einer Luftaustrittstemperatur von 40 bis 170°C getrocknet wird.

3. Verfahren gemäß Anspruch 1 zur Herstellung von sauren Brausekomponenten mit einer mittleren Teilchengröße von 100 bis 190 μm und einem Schüttvolumen von 130 bis 270 ml/100 g.

4. Verfahren gemäß Anspruch 1, dadurch gekennzeichnet, daß die eingesetzte Speiseflüssigkeit 30 bis 50 % Feststoffanteile enthält.

5. Verfahren gemäß Anspruch 1, dadurch gekennzeichnet, daß die Sprühtrocknung bei einer Lufteintrittstemperatur von 120 bis 210°C und einer Luftaustrittstemperatur von 50 bis 140°C durchgeführt wird.

6. Verfahren gemäß Ansprüchen 1 bis 5, dadurch gekennzeichnet, daß die Lösung oder Suspension der sauren Salze zusammen mit gegebenenfalls vorhandenen weiteren Zuschlagstoffen sprühgetrocknet wird.

7. Verfahren zur Herstellung von Brausetabletten, dadurch gekennzeichnet, daß man die Brausekomponenten gemäß Anspruch 1 nach üblichen Methoden direkttablettiert.

**Claims**

**Claims for the following Contracting States : AT, BE, CH, LI, DE, DK, FR, GB, IT, LU, NL, SE**

1. Acid spray-dried effervescent component which is essentially spherical and comprises an acid salt of tartaric and/or citric acid.

2. Effervescent component according Claim 1, which essentially comprises mono- or disodium citrate, mono- or dipotassium citrate, mono- or diammonium citrate, monosodium, monopotassium or monoammonium tartrate or mixtures of these salts.

3. Effervescent component according Claim 1, essentially comprising a salt of citric acid.

4. Effervescent component according Claims 1 to 3, characterized in that it essentially comprises spherical individual particles which have an average particle size of 70 to 250 μm and a bulk volume of 110 to 350 ml/100 g.

5. Effervescent component according Claim 4 having an average particle size of 100 to 190 μm and a bulk volume of 130 to 270 ml/100g.

6. Process for the preparation of acid spray-dried effervescent components which can be tableted directly and essentially comprise spherical particles of an acid salt of tartaric and/or citric acid, characterized in that the acid salts are spray dried by customary methods.

7. Spray drying process according to Claim 6, characterized in that a solution or suspension of the acid salts of tartaric and/or citric acid in a suitable solvent or solvent mixture is first prepared as an edible liquid which comprises a solids content of 10 to 70 % and this edible liquid is then dried in a spray drier at an air intake temperature of 100 to 250°C and an air outlet temperature of 40 to 170°C.

8. Process according to Claim 6, characterized in that the edible liquid employed comprises solids contents of 30 to 50%.

9. Process according to Claim 6, characterized in that the spray drying is carried out at an air intake temperature of 120 to 210°C and an air outlet temperature of 50 to 140°C.

10. Process according to Claims 6 to 8, characterized in that the solution or suspension of the acid salts is spray dried together with any other additives which may be present.

11. Use of effervescent components according to Claim 1 for the preparation of effervescent formulations in the form of effervescent tablets, effervescent granules or effervescent powders.

12. Process for the preparation of effervescent tablets, characterized in that the effervescent component according to Claim 1 is tableted directly by customary methods.

**Claims for the following Contracting States : ES, GR**

1. Process for the preparation of acid spray-dried effervescent components which can be tableted directly and essentially comprise spherical particles of an acid salt of tartaric and/or citric acid, characterized in that the acid salts are spray dried by customary methods.

2. Spray drying process according to Claim 1, characterized in that a solution or suspension of the acid salts of tartaric and/or citric acid in a suitable solvent or solvent mixture is first prepared as an edible liquid which comprises a solids content of 10 to 70 % and this edible liquid is then dried in a spray drier at an air intake temperature of 100 to 250°C and an air outlet temperature of 40 to 170°C.

3. Process according to Claim 1 for the preparation of acid effervescent components having an average particle size of 100 to 190 μm and a bulk volume of 130 to 270 ml/100g.

4. Process according to Claim 1, characterized in that the edible liquid employed comprises solids contents of 30 to 50%.

5. Process according to Claim 1, characterized in that the spray drying is carried out at an air intake temperature of 120 to 210°C and an air outlet temperature of 50 to 140°C.

6. Process according to Claims 1 to 5, characterized in that the solution or suspension of the acid salts is spray dried together with any other additives which may be present.

7. Process for the preparation of effervescent tablets, characterized in that the effervescent components according to Claim 1 are tableted directly by customary methods.

**Revendications**

**Revendications pour les Etats contractants suivants : AT, BE, CH, LI, DE, DK, FR, GB, IT, LU, NL, SE**

1. Composant effervescent acide séché par pulvérisation essentiellement sous forme de particules sphériques et contenant un sel acide de l'acide tartrique et/ou de l'acide citrique.

2. Composant acide selon la revendication 1, contenant essentiellement du citrate mono- ou bisodique, du citrate mono- ou bipotassique, du citrate mono- ou biammonique, du tartrate monosodique, monopotassique ou monoammonique ou des mélanges de ces sels.

3. Composant effervescent selon la revendication 1, contenant essentiellement un sel de l'acide citrique.

4. Composant effervescent selon les revendications 1 à 3, caractérisé en ce qu'il est constitué essentiellement de particules sphériques ayant une granulométrie moyenne de 70 à 250 μm et un volume en vrac de 110 à 350 ml/100 g.

5. Composant effervescent selon la revendication 4, avec une granulométrie moyenne de 100 à 190 μm et un volume en vrac de 130 à 270 ml/100 g.

6. Procédé de fabrication de composants effervescents acides séchés par pulvérisation pouvant être transformés directement en tablettes et formés de particules essentiellement sphériques contenant un sel acide de l'acide tartrique et/ou de l'acide citrique, caractérisé en ce que les sels acides sont séchés par pulvérisation par des méthodes usuelles.

7. Procédé de séchage par pulvérisation selon la revendication 6, caractérisé en ce qu'une solution ou une suspension du sel acide de l'acide tartrique et/ou de l'acide citrique est tout d'abord mise sous forme de liquide d'alimentation en utilisant un solvant ou un mélange de solvants appropriés, de façon à obtenir une teneur en substances solides de 10 à 70%, ce liquide d'alimentation étant ensuite séché dans un sécheur par pulvérisation avec une température d'entrée d'air de 100 à 250°C et une température de sortie d'air de 40 à 170°C.

8. Procédé selon la revendication 6, caractérisé en ce que le liquide d'alimentation utilisé contient de 30 à 50% de matières solides.

9. Procédé selon la revendication 6, caractérisé en ce que le séchage par pulvérisation est effectué avec une température d'entrée d'air de 120 à 210°C et une température de sortie d'air de 50 à 140°C.

10. Procédé selon les revendications 6 à 8, caractérisé en ce que la solution ou la suspension du sel acide est séchée par pulvérisation en même temps que les additifs éventuellement présents.

11. Utilisation du composant effervescent selon la revendication 1 pour la fabrication de préparations effervescentes sous forme de tablettes effervescentes, de granulés effervescents ou de poudres effervescentes.

12. Procédé de fabrication de tablettes effervescentes, caractérisé en ce que l'on transforme directement en tablettes le composant effervescent selon la revendication 1 en utilisant les méthodes usuelles.

**Revendications pour les Etats contractants suivants : ES, GR**

1. Procédé de fabrication de composants effervescents acides séchés par pulvérisation pouvant être transformés directement en tablettes et qui contiennent des particules essentiellement sphériques d'un sel acide de l'acide tartrique et/ou de l'acide citrique, caractérisé en ce que les sels acides sont séchés par pulvérisation en utilisant les procédés usuels.

2. Procédé de séchage par pulvérisation selon la revendication 1, caractérisé en ce que l'on prépare tout d'abord une solution ou une suspension du sel acide de l'acide tartrique et/ou de l'acide citrique dans un solvant ou un mélange de solvants appropriés, de façon à obtenir un liquide d'alimentation contenant une teneur en matières solides de 10 à 70%, ce liquide d'alimentation étant ensuite séché dans un sécheur par pulvérisation avec une température d'entrée d'air de 100 à 250°C et une température de sortie d'air de 40 à 170°C.

3. Procédé selon la revendication 1 pour la préparation de composants effervescents acides ayant une granulométrie moyenne de 100 à 190 µm et un volume en vrac de 130 à 270 ml/100 g.

4. Procédé selon la revendication 1, caractérisé en ce que le liquide d'alimentation utilisé contient de 30 à 50% de matières solides.

5. Procédé selon la revendication 1, caractérisé en ce que le séchage par pulvérisation est effectué avec une température d'entrée d'air de 120 à 210°C et une température de sortie d'air de 50 à 140°C.

6. Procédé selon les revendications 1 à 5, caractérisé en ce que la solution de la suspension du sel acide est séchée par pulvérisation avec les autres additifs éventuellement présents.

7. Procédé de fabrication de tablettes effervescentes, caractérisé en ce que le composant effervescent selon la revendication 1 est transformé directement en tablettes en utilisant les méthodes usuelles.